(19) Europäisches Patentamt
European Patent Office
Office européen des brevets

(11) Numéro de publication: **0 199 641**
**B1**

# (12) FASCICULE DE BREVET EUROPÉEN

(45) Date de publication du fascicule du brevet: **21.09.88**

(21) Numéro de dépôt: **86400815.6**

(22) Date de dépôt: **16.04.86**

(51) Int. Cl.⁴: **C 07 D 487/04**, C 07 D 471/04, C 07 D 471/18, A 61 K 31/495 // (C07D487/04, 249:00, 209:00), (C07D471/04, 249:00, 221:00), (C07D471/14, 249:00, 221:00, 221:00)

## (54) Nouveaux dérivés du triazole, leur procédé de préparation et les compositions pharmaceutiques les renfermant.

(30) Priorité: **17.04.85 FR 8505762**

(43) Date de publication de la demande: **29.10.86 Bulletin 86/44**

(45) Mention de la délivrance du brevet: **21.09.88 Bulletin 88/38**

(84) Etats contractants désignés: **AT BE CH DE FR GB IT LI LU NL SE**

(56) Documents cité:
**GB-A-2 020 269**

**CHEMICAL ABSTRACTS, vol. 97, 1982, page 59, abrégé 66296k, Columbus, Ohio, US; B. SILVESTRINI et al.: "Psychopharmacological profile of dapiprazole, a new potential antipsychotic agent"**

(73) Titulaire: **ADIR ET COMPAGNIE, 22, rue Garnier, F-92200 Neuilly- sur- Seine (FR)**

(72) Inventeur: **Wierzbicki, Michel, 32 rue Lucien Voilin, F-92800 Puteaux (FR)**
Inventeur: **Hugon, Pierre, 13 rue Eugène Sue, F-92500 Reuil- Malmaison (FR)**
Inventeur: **Poignant, Jean- Claude, "La Guyonnerie" 13 rue de la Fontaine St- Mathieu, F-91440 Bures- sur- Yvette (FR)**

(74) Mandataire: **Reverbori, Marcelle, ADIR 22 Rue Garnier, F-92200 Neuilly- sur- Seine (FR)**

EP 0 199 641 B1

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

LIBER, STOCKHOLM 1988

**Description**

La présente invention a pour objet de nouveaux dérivés du triazole, leur procédé de préparation et les compositions pharmaceutiques les renfermant.

Elle concerne particulièrement les dérivés du triazole de formule générale I:

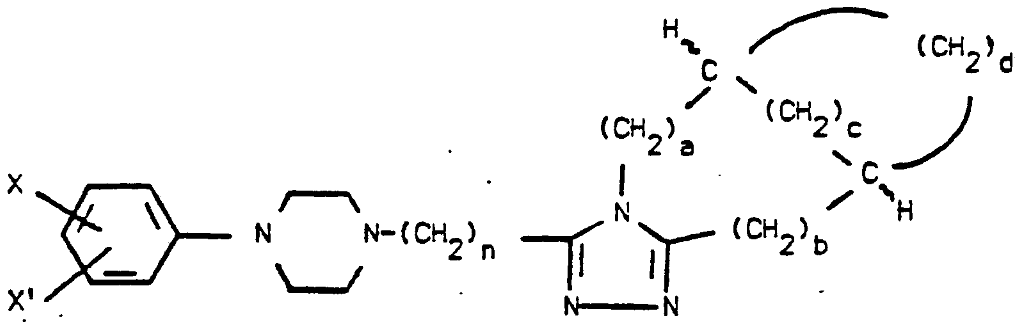

dans laquelle:

- X et X', identiques ou différents représentent chacun un atome d'hydrogène ou d'halogène, un radical trifluorométhyle ou un radical alcoyle ou alcoxy ayant chacun de 1 à 5 atomes de carbone, ou
- X et X' représentent ensemble un radical méthylène dioxy;
- n représente un nombre entier de 1 à 6;
- a représente les valeurs zéro, 1 ou 2;
- b représente les valeurs zéro, 1, 2 ou 3;
- c représente les valeurs zéro, 1 ou 2 et
- d'représente les valeurs 2, 3, 4 ou 5 de telle sorte que la somme: a + b + c + d soit égale à 4, 5, 6 ou 7.

L'état antérieur de la technique dans ce domaine est illustré notamment par les brevets allemands N° 2 915 318 et belge N° 877 161 qui ont pour objet des dérivés du cycloalkyl triazole de formule générale :

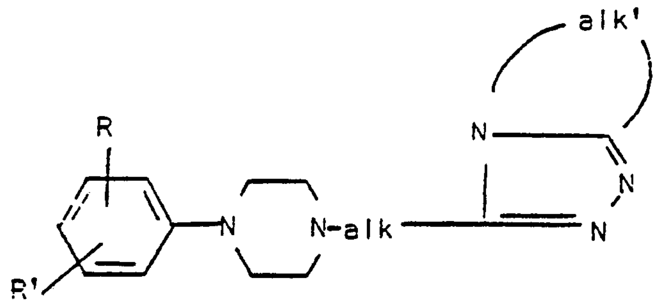

dans laquelle:

- alk représente une chaîne aliphatique bivalente contenant de 1 à 10 atomes de carbone
- alk' représente une chaîne aliphatique lineaire ou ramifiée contenant de 1 à 5 atomes de carbone, et R et R' peuvent être hydrogène, halogène, alcoyle, alcoxy, hydroxy, trifluorométhyle ou méthylthio.

Ces dits composés sont mentionnés comme agents antiglaucomateux et antipsychotiques.

L'introduction d'une partie alicyclique condensée sur le cycle mono azoté de ces composés a conduit à l'obtention des dérivés de formule générale I, et a permis de mettre en évidence, pour ces dérivés (I), une activité analgésique ou anxiolytique remarquable nullement mentionnée pour les composés analogues de l'état antérieur.

La présente invention a également pour objet le procédé de préparation des dérivés de formule générale I, caractérisé en ce que:

- l'on fait réagir un composé de formule générale II:

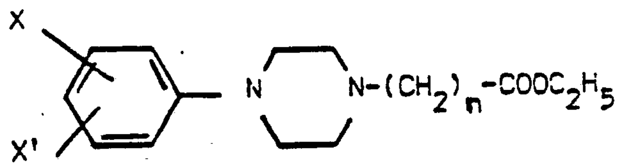

(II)

dans laquelle X, X' et n ont les significations précédemment définies, avec l'hydrazine $H_2N-NH_2$, pour obtenir un composé de formule générale III:

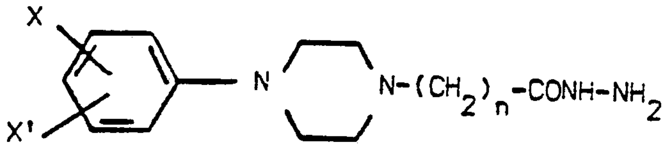

(III)

dans laquelle X, X' et n sont tels que précédemment définis,

- puis l'on fait réagir ce composé (III) avec un lactime éther de formule générale IV:

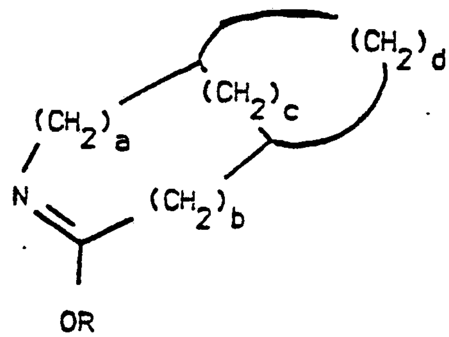

(IV)

dans laquelle a, b, c et d sont tels que précédemment définis et R représente un radical alcoyle ayant de 1 à 5 atomes de carbone, de préférence un radical méthyle.

La préparation des composés III s'effectue avantageusement en faisant réagir un composé II avec l'hydrate d'hydrazine dans un solvant convenablement choisi, tel que par exemple un alcool.

La réaction des composés III avec les lactimes éthers IV s'effectue de manière particulièrement convenable dans un solvant adéquat, choisi par exemple parmi les hydrocarbures aromatiques éventuellement halogénés en distillant éventuellement les produits formés (alcools, eau...).

Les lactimes éthers de formule generale IV sont eux-mêmes préparés par action d'agents alcoylants, tels que par exemple le sulfate de méthyle, sur les lactames correspondants de formule générale IVa:

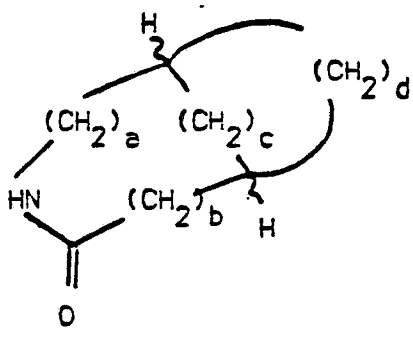

(IVa)

dans laquelle a, b, c et d sont tels que précédemment définis;

lesquels lactames sont des produits décrits dans la littérature.

Les produits de départ de formule générale II sont eux-mêmes préparés à partir des pipérazines N-monosubstituées de formule générale IIa:

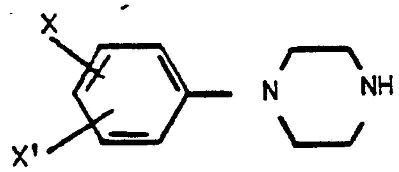

(IIa)

dans laquelle X et X' sont tels que précédemment définis, lesquelles sont des produits connus de la littérature, que l'on fait réagir avec un dérivé halogéné de formule générale IIb:

$$Hal - A- COOC_2H_5 \quad (IIb)$$

dans laquelle:

- Hal représente un atome d'halogène, tel que par exemple un atome de brome ou de chlore; et
- A représente une chaîne hydrocarbonée contenant n atomes de carbone (n étant tel que précédemment défini) et renfermant éventuellement, dans le cas où n est supérieur à 1, une double liaison en position α de la fonction carboxyéthyle.

La condensation des composés IIa et IIb s'effectue avantageusement dans un solvant adéquat tel que par exemple, le benzène, l'acétone, le diméthylformamide ou le dioxanne, en opérant éventuellement en présence d'une base qui peut être un hydrure, un alcoolate ou un carbonate alcalin, qui sert d'accepteur de l'hydracide formé au cours de la réaction.

Cette condensation étant suivie, dans le cas où A renferme une double liaison, d'une hydrogénation catalytique de l'ester insaturé ainsi obtenu.

Les produits de départ de formule générale II dans laquelle n est un nombre entier supérieur à 1, et qui de ce fait répondent à la formule générale II':

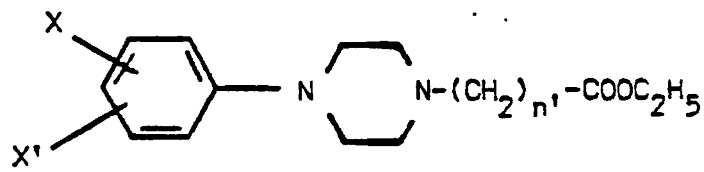

(II')

dans laquelle X et X' sont tels que précédemment définis et n'représente un nombre entier de 2 à 6,

peuvent également être préparés en faisant réagir les pipérazines N-monosubstituées de formule générale IIa, précédemment définie, avec un dérivé de formule générale II'b:

$$H_2C = CH - (CH_2)_{n'-2} - COOC_2H_5 \quad (II'b)$$

dans laquelle n' représente un nombre entier de 2 à 6.

La condensation s'effectue avantageusement dans un solvant approprié choisi, par exemple, parmi les alcools et les hydrocarbures aromatiques.

Ce dernier procédé est particulièrement adéquat pour préparer les dérivés de formule générale II dans laquelle n est égal à 2.

Les dérivés (I) ainsi obtenus peuvent être transformés en sels d'addition avec les acides, sels qui font, à ce titre, partie de la présente invention. Comme acides utilisables pour la formation de ces sels, on peut citer, dans la série minérale: les acides chlorhydrique, bromhydrique, sulfurique, phosphorique, et dans la série organique: les acides acétique, propionique, maléique, fumarique, tartrique, citrique, oxalique, benzoïque et méthane sulfonique.

Les dérivés de formule générale I et leurs sels physiologiquement tolérables possèdent des propriétés pharmacologiques et thérapeutiques intéressantes notamment des propriétés anxiolytique ou analgésique; qui de ce fait, permettent leur utilisation comme médicament notamment dans le traitement de la douleur et des états anxieux.

La présente invention a également pour objet les compositions pharmaceutiques contenant comme principe actif un composé de formule générale I ou un de ses sels physiologiquement tolérable, mélangé ou associé à un excipient pharmaceutique approprié, comme par exemple, l'eau distillée, le glucose, le lactose, l'amidon, le talc, l'éthyl cellulose, le stéarate de magnésium ou le beurre de cacao.

Les compositions pharmaceutiques ainsi obtenues se présentent généralement sous forme dosée et peuvent contenir de 3 à 300 mg de principe actif. Elles peuvent revêtir, par exemple, la forme de comprimés, dragées, gélules, suppositoires, solutions injectables ou buvables, et être selon les cas administrés par voie orale, rectale ou parentérale à la dose de 3 à 300 mg, 1 à 3 fois par jour.

Les exemples suivants illustrent l'invention, les points de fusion étant, sauf mention contraire, déterminés à la platine chauffante de Kofler.

**Exemple 1:**

N'- [N-(métachlorophényl)pipérazine] méthyl-8 hexahydro-2b,3,4,5,5a,6 triazolo [3,4-a] cyclopenta (c) pyrrole

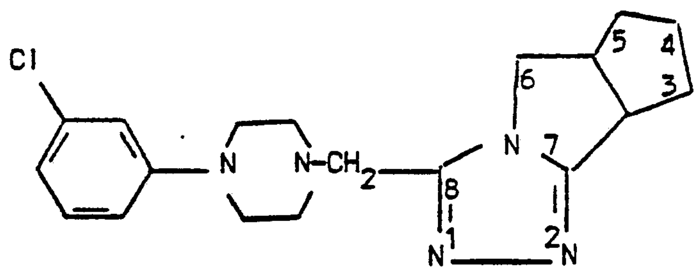

8 g d'hydrazide de l'acide [(chloro-3 phényl)-4 pipérazinyl-1] acétique et 4,6 g de méthoxy-2 aza-3 bicyclo (3,3,0) octène-2 dissous dans 80 ml de xylène sont chauffés à reflux pendant 20 heures en distillant à 5, 10 et 15 heures quelques millilitres afin d'éliminer les produits légers (eau et méthanol) formés dans la réaction. Le mélange réactionnel est ensuite évaporé à sec, sous vide et le résidu est trituré avec 100 ml d'éther anhydre. Après essorage, le produit obtenu (7,6 g; P.F.: 128°C) est cristallisé dans 45 ml d'acétate d'éthyle bouillant. Après refroidissement, essorage, séchage, on obtient 4 g de N'-[N-métachlorophényl)pipérazine] méthyl-8 hexahydro-2b, 3,4,5,5a,6 triazolo [3,4-a] cyclopenta (c) pyrrole, P.F.: 155°C.

Les matières premières ont été préparées comme suit:

A/ Préparation de l'hydrazide de l'acide [(chloro-3 phényl)-4 pipérazinyl] -1 acétique.

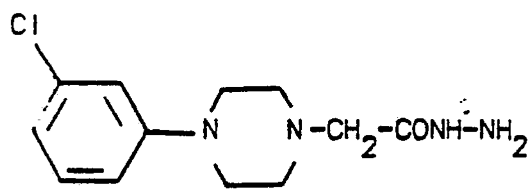

a) éthyl ester de l'acide [(chloro-3 phényl)-4 pipérazinyl-1] acétique.

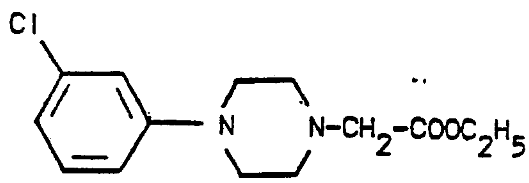

33,4 g de bromoacétate d'éthyle sont ajoutés en 20 minutes dans une solution de 78,7 g de N-(chloro-3 phényl) pipérazine dans 400 ml de toluène anhydre, puis le mélange réactionnel est maintenu à reflux pendant 45 minutes. Le bromhydrate de la pipérazine de départ est essoré et le filtrat est concentré à sec sous vide. Le produit brut obtenu est utilisé tel quel dans la phase suivante.

De la même façon ont été préparés:

- l'éthyl ester de l'acide [(trifluorométhyl-3 phényl)-4 pipérazinyl-1] acétique, et
- l'éthyl ester de l'acide [(dichloro-3,5 phényl)-4 pipérazinyl-1] acétique.

b) hydrazide de l'acide [(chloro-3 phényl)-4 pipérazinyl-1] acétique.

56,5 g d'éthyl ester de l'acide (chloro-3 phényl)-4 pipérazinyl-1 acétique et 50 g d'hydrate d'hydrazine sont dissous dans 400 ml d'éthanol. Le mélange réactionnel est maintenu à reflux pendant 1 heure 30 minutes. Après évaporation sous vide des produits légers, l'hydrazide restant est utilisé tel quel pour la synthèse suivante.

De la même façon, ont été préparés:

- l'hydrazide de l'acide [(trifluorométhyl-3 phényl)-4 pipérazinyl-1] acétique; et
- l'hydrazide de l'acide [(dichloro-3,5 phényl)-4 pipérazinyl-1] acétique.

B/ méthoxy-2 aza-3 bicyclo (3,3,0) octène-2:

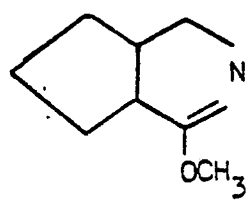

A une solution maintenue à reflux de 125,2 g d'oxo-2 aza-3 bicyclo (3,3,0) octane dans 350 ml de benzène anhydre sont ajoutés goutte à goutte en 1 heure 94,7 ml de sulfate de diméthyle. Le reflux est ensuite maintenu pendant 4 heures. Après refroidissement, le mélange réactionnel est versé sur 100 ml de lessive de soude concentrée. La couche organique est décantée, la couche aqueuse est extraite par 2 fois 200 ml de benzène. Les extraits benzéniques sont réunis, séchés sur sulfate de magnésium, concentrés sous vide et le résidu est distillé. On obtient ainsi 79,5 g de méthoxy-2 aza-3 bicyclo (3,3,0) octène-2, PEb: 64 - 68°C, $n^{23}_D$ = 1,4718.

De la même façon, ont été préparés les lactimes éthers de formule:

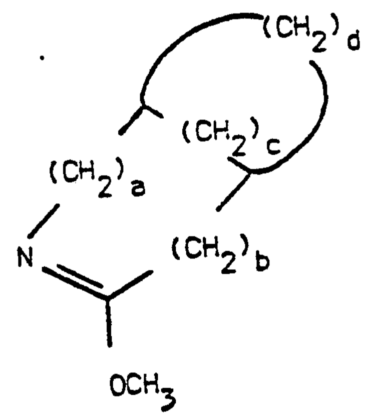

dont les caractéristiques sont regroupées dans le tableau ci-après:

| a | b | c | d | Forme | Caractéristiques physiques |
|---|---|---|---|---|---|
| 1 | 0 | 0 | 4 | - | $PEb/_{9mm\ Hg}$ = 83 - 85°C<br>$n^{23}_D$ = 1,4772 |
| 1 | 1 | 0 | 4 | cis | Produit brut |
| 1 | 1 | 0 | 4 | trans | Produit brut |
| 0 | 2 | 0 | 4 | cis | Produit brut |
| 0 | 2 | 0 | 4 | trans | Produit brut |

**Exemple 2:**

β-N'-[N-(métachlorophényl)pipérazine] éthyl-9 [7H] hexahydro-2b,3,4,5,6,6a triazolo [3,4-a] cyclo hexa (c) pyrrole

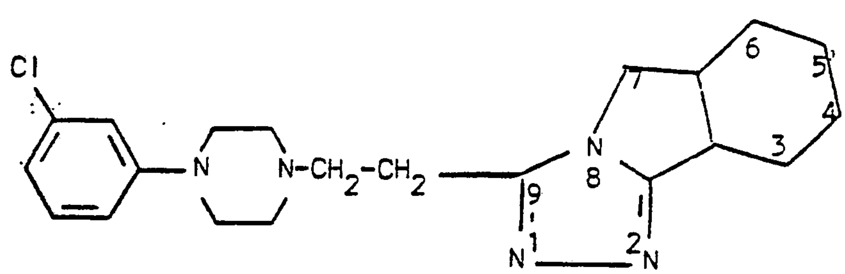

14 g d'hydrazide de l'acide [(choro-3 phényl)-4 pipérazinyl-1] propionique et 8,4 g de méthoxy-2 aza-3 bicyclo (4,3,0) nonène-2 sont chauffés 1 heure dans 120 ml de trichloro-1,2,4 benzène. Après élimination du solvant sous vide, le résidu est recristallisé dans 50 ml d'acétate d'éthyle. On obtient 4,6 g de β-N'-[N(métachloro-phényl) pipérazine] éthyl-9[7H] hexahydro-2b,3,4,5,6,6a, triazolo [3,4-a] cyclo hexa (c) pyrrole, P.F.: 152 - 153°C.

L'hydrazide de l'acide [(chloro-3 phényl)-4 pipérazinyl-1] propionique de départ, P.F.: 113 - 114°C (acide acétique), a été préparé selon le mode opératoire décrit dans l'exemple 1, à partir de l'éthyl ester de l'acide [(chloro-3 phényl)-4 pipérazinyl-1] propionique, lui-même préparé comme suit.

A 38,3 g de N-(chloro-3 phényl) pipérazine dissous dans 300 ml d'éthanol anhydre et 0,5 ml de Triton B sont ajoutés en 40 minutes 108 ml d'acrylate d'éthyle. La température atteint 40° C en fin de coulée. Après 3 heures de chauffage à reflux, le mélange réactionnel est concentré sous vide et le résidu distillé. On obtient 139 g d'éthyl ester de l'acide [(chloro-3 phényl)-4 pipérazinyl-1] propionique, $PEb/_{0,3\ mm\ Hg}$ = 176 - 179° C.

De la même façon, ont été préparés les esters dont les caractéristiques sont regroupées dans le tableau ci-après:

formule:

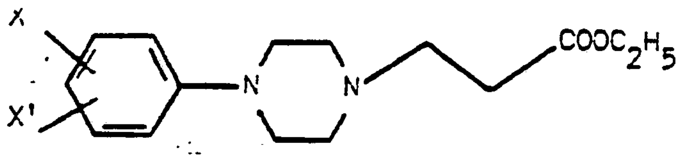

| X | X' | Constantes physiques |
|---|---|---|
| (2)-Cl | H | $PEb/_{0,15\ mm\ Hg}$ = 165 - 166° C |
| (4)-Cl | H | P.F.: 56 - 57° C |
| (3)-$CF_3$ | H | liquide brut |
| (3)-F | H | $PEb/_{0,1\ mm\ Hg}$ = 155 - 157° C |
| (3)-$CH_3$ | H | $PEb/_{0,15\ mm\ Hg}$ = 153 - 156° C |
| (3)-$OCH_3$ | H | $PEb/_{0,15\ mm\ Hg}$ = 182 - 184° C |
| (3)-Cl | (4)-Cl | liquide brut |
| (3)-Cl | (5)-Cl | PF: 74° C (cyclohexane) |
| (3)-$OCH_3$ | (4)-$OCH_3$ | PF: 48° C |
| (3)-$CF_3$ | (4)-Cl | $PEb/_{0,4\ mm\ Hg}$ = 162 - 168° C |
| (3)-$CF_3$ | (4)-F | $PEb/_{0,3\ mm\ Hg}$ = 156° C |
| (3,4)-O-$CH_2$-O- | | PF: 50° C |

A partir de ces esters, ont été préparés les hydrazides corresondants de formule:

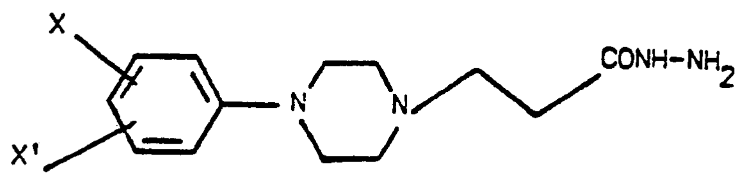

dont les caractéristiques sont regroupées dans le tableau ci-après.

| X | X' | Constantes physiques |
|---|---|---|
| (2)-Cl | H | P.F.: 132 - 133° C (isopropanol) |
| (4)-Cl | H | P.F.: 154° C (isopropanol) |
| (3)-$CF_3$ | H | P.F.: 99° C ($C_6H_6$/éther de pétrole) |
| (3)-F | H | P.F.: 130° C (isopropanol) |
| (3)-$CH_3$ | H | P.F.: 98° C (isopropanol) |
| (3)-Cl | (4)-Cl | P.F.: 88 - 90° C (benzène) |
| (3)-Cl | (5)-Cl | P.F.: 136° C (isopropanol) |
| (3)-$CF_3$ | (4)-Cl | liquide brut |
| (3)-$CF_3$ | (4)-F | P.F.: 96° C ($CH_3COOH$/éther de pétrole) |
| (3)-$OCH_3$ | H | P.F.: 121 - 122° C (isopropanol) |
| (3)-$OCH_3$ | (4)-$OCH_3$ | P.F.: 142° C (isopropanol) |
| (3,4)-O-$CH_2$-O- | | P.F.: 142° C (isopropanol) |

**Exemple 3:**

cis γ-N [N'-(métatrifluorométhylphényl) pipérazine] n.propyl-10 octahydro-3,3a,4,5,6,7,7a,8 triazolo [4,3-b] isoquinoléine.

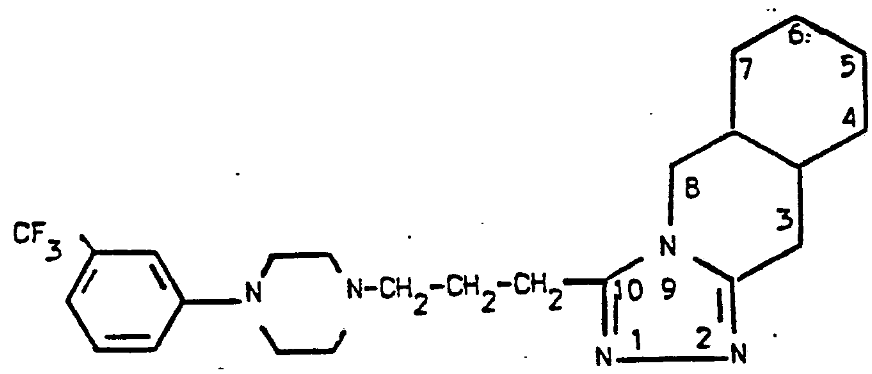

A partir de 11 g d'hydrazide de l'acide [(trifluorométhyl-3 phényl)-4 pipérazinyl-1] butanoïque et 7,5 g de méthoxy-4 aza-3 bicyclo (4,4,0) decène-3 (forme cis) dans 75 ml de xylène, en opérant comme dans l'exemple 1, puis en traitant le résidu, après élimination du solvant par 100 ml d'isopropanol anhydre et 27 ml d'éther chlorhydrique 2,44N, on obtient 4,7 g de dichlorhydrate de cis γ-N[N'-(métatrifluorométhyl-phényl) pipérazine] n.propyl-10-octahydro-3,3a,4,5,6,7,7a,8 triazolo [4,3-b] isoquinoléine, P.F.: 210 - 220°C (isopropanol).

L'hydrazide de l'acide [(trifluorométhyl-3 phényl)-4 pipérazinyl-1] butanoïque de départ, a été préparé en opérant comme dans l'exemple 1, à partir de l'éthyl ester de l'acide [(trifluorométhyl-3 phényl)-4 pipérazinyl-1] butanoïque lui-même préparé comme suit:

A 69 g de (trifluorométhyl-3 phényl)-1 pipérazine en solution dans 300 ml de toluène anhydre sont ajoutés en 20 minutes 29 g de bromo-4 crotonate d'éthyle. Le mélange est ensuite chauffé à reflux 1 heure. Après refroidissement le bromhydrate est essoré et le filtrat concentré à sec sous vide. On obtient ainsi à l'état brut avec un rendement théorique, l'éthyl ester de l'acide [(trifluorométhyl-3 phényl)-4 pipérazinyl-1] butène-2 oïque.

23 g de cet ester dissous dans 200 ml d'éthanol sont hydrogénés sous 60.LBS de pression en présence de nickel de Raney. La quantité théorique d'hydrogène est absorbée en 3 heures. Après filtration du catalyseur, le filtrat est concentré sous vide et le résidu distillé. On obtient ainsi 31 g d'éthyl ester de l'acide [(trifluorométhyl-3 phényl)-4 pipérazinyl-1] butanoïque, $PEb/_{0,3\ mm\ Hg}$ = 146 - 147°C.

De la même façon ont été préparés l'éthyl ester de l'acide [(chloro-3 phényl)-4 pipérazinyl-1] butanoïque, et l'éthyl ester de l'acide [(dichloro-3,5 phényl)-4 pipérazinyl-1] butanoïque, qui sont chacun des liquides obtenus et utilisés à l'état brut pour préparer respectivement: l'hydrazide de l'acide (chloro-3 phényl)-4 pipérazinyl-1 butanoïque, P.F.: 45 - 50°C (éther) et de l'hydrazide de l'acide (dichloro-3,5 phényl)-4 pipérazinyl-1 butanoïque, P.F.: 136°C (isopropanol).

**Exemple 4 - 56:**

En opérant comme dans les exemples précédents, ont éte préparés les produits dont les caractéristiques sont regroupées dans le tableau ci-après.

Formule:

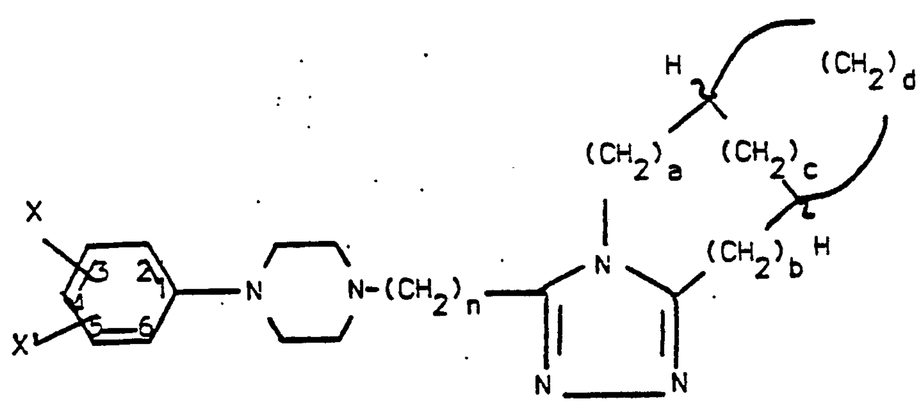

| Ex | X | X' | n | a | b | c | d | Jonction de cycle | Constantes physiques |
|---|---|---|---|---|---|---|---|---|---|
| 4 | (3)$CF_3$ | H | 1 | 1 | 1 | 0 | 4 | trans | P.F.: 143°C base |
| 5 | (3)Cl | H | 1 | 0 | 2 | 0 | 4 | trans | P.F.: 161 - 162°C base |
| 6 | (3)Cl | H | 1 | 2 | 0 | 0 | 4 | cis | P.F.: 174°C base |
| 7 | (3)Cl | H | 1 | 0 | 2 | 0 | 4 | cis | P.F.: 173 - 174°C base |
| 8 | (3)$CF_3$ | H | 1 | 1 | 0 | 0 | 3 | | P.F.: 128°C base |
| 9 | (3)Cl | (5)Cl | 1 | 0 | 2 | 0 | 4 | cis | P.F.: 166 - 167°C base |
| 10 | (3)Cl | (5)Cl | 1 | 0 | 2 | 0 | 4 | trans | P.F.: 180°C base |
| 11 | (3)Cl | H | 2 | 1 | 1 | 0 | 4 | cis | P.F.: 130°C base |
| 12 | (3)Cl | H | 2 | 0 | 2 | 0 | 4 | trans | P.F.: 162°C base |
| 13 | (3)Cl | H | 2 | 1 | 0 | 0 | 3 | | P.F.: 156°C base |
| 14 | (3)Cl | H | 2 | 1 | 1 | 0 | 4 | trans | P.F.: 132°C base |
| 15 | (3)$CF_3$ | H | 2 | 0 | 2 | 0 | 4 | trans | P.F.: 152°C base |
| 16 | (3)$CF_3$ | H | 2 | 1 | 1 | 0 | 4 | cis | P.F.: 144°C base |
| 17a | (3)$CF_3$ | H | 2 | 1 | 0 | 0 | 3 | | P.F.: 138°C base |
| 17b | (3)$CF_3$ | H | 2 | 1 | 0 | 0 | 3 | | P.F.: 168°C méthane sulfonate |
| 17c | (3)$CF_3$ | H | 2 | 1 | 0 | 0 | 3 | | P.F.: 262°C (dec) monochlorhydrate |
| 17d | (3)$CF_3$ | H | 2 | 1 | 0 | 0 | 3 | | P.F.: 240°C dichlorhydrate |
| 18 | (3)Cl | H | 2 | 2 | 0 | 0 | 4 | cis | P.F.: 183 - 184°C base |
| 19 | (3)$CF_3$ | H | 2 | 2 | 0 | 0 | 4 | cis | P.F.: 176°C base |
| 20 | (3)Cl | H | 2 | 0 | 0 | 2 | 2 | | P.F.: 116°C base |
| 21 | (3)$CF_3$ | H | 2 | 0 | 0 | 2 | 2 | | P.F.: 136°C base |
| 22 | (3)$OCH_3$ | H | 2 | 0 | 2 | 0 | 4 | trans | P.F.: 137 - 138°C base |
| 23 | (3)$CF_3$ | (4)F | 2 | 0 | 2 | 0 | 4 | trans | P.F.: 185°C base |
| 24 | (3)$OCH_3$ | (4)$OCH_3$ | 2 | 0 | 2 | 0 | 4 | trans | P.F.: 145°C base |
| 25 | (4)Cl | H | 2 | 0 | 2 | 0 | 4 | trans | P.F.: 170 - 171°C base |
| 26 | (3)F | H | 2 | 0 | 2 | 0 | 4 | trans | P.F.: 135 - 136°C base |
| 27 | (3)$CF_3$ | (4)Cl | 2 | 0 | 2 | 0 | 4 | trans | P.F.: 180°C base |
| 28 | (3)Cl | (5)Cl | 2 | 0 | 2 | 0 | 4 | trans | P.F.: 176°C base |
| 29 | (3)$CH_3$ | H | 2 | 0 | 2 | 0 | 4 | trans | P.F.: 146°C base |
| 30 | (2)Cl | H | 2 | 0 | 2 | 0 | 4 | trans | P.F.: 157 - 158°C base |
| 31 | (3)Cl | (4)Cl | 2 | 0 | 2 | 0 | 4 | trans | P.F.: 192°C base |
| 32 | (3,4)O-$CH_2$-O | | 2 | 0 | 2 | 0 | 4 | trans | P.F.: 125°C base |
| 33 | (3)$CF_3$ | (4)Cl | 2 | 0 | 2 | 0 | 4 | cis | P.F.: 173°C base |
| 34 | (3)Cl | H | 2 | 0 | 2 | 0 | 4 | cis | P.F.: 145 - 146°C base |
| 35 | (3)$OCH_3$ | (4)$OCH_3$ | 2 | 0 | 2 | 0 | 4 | cis | P.F.: 135°C base |
| 36 | (3)$CF_3$ | H | 2 | 1 | 0 | 0 | 4 | | P.F.: 140°C base |
| 37 | (3)Cl | (5)Cl | 2 | 0 | 2 | 0 | 4 | cis | P.F.: 215 - 232°C dichlorhydrate |
| 38 | (3)Cl | (5)Cl | 2 | 1 | 0 | 0 | 3 | | P.F.> 260°C base dichlorhydrate |
| 39 | (3)Cl | (5)Cl | 2 | 1 | 1 | 0 | 4 | cis | P.F.: 162 - 164°C base |
| 40 | (3)Cl | H | 3 | 1 | 1 | 0 | 4 | cis | P.F.> 260°C dichlorhydrate |
| 41 | (3)Cl | (5)Cl | 3 | 0 | 2 | 0 | 4 | trans | P.F.: 149°C base |
| 42 | (3)Cl | H | 3 | 0 | 2 | 0 | 4 | cis | P.F.: 210 - 215°C dichlorhydrate |
| 43 | (3)Cl | (5)Cl | 3 | 0 | 2 | 0 | 4 | cis | P.F.: 137°C base |
| 44 | (4)$CF_3$ | H | 3 | 1 | 1 | 0 | 4 | cis | P.F.: 250 - 255°C dichlorhydrate |
| 45 | (3)$CF_3$ | H | 3 | 1 | 0 | 0 | 3 | | P.F.: 230°C trichlorhydrate |
| 46 | (3)$CF_3$ | H | 3 | 0 | 2 | 0 | 4 | trans | P.F.: 210 - 212°C dichlorhydrate |
| 47 | (2)$CF_3$ | H | 3 | 1 | 1 | 0 | 4 | cis | P.F.: 242 - 246°C dichlorhydrate |
| 48 | (3)Cl | H | 3 | 0 | 2 | 0 | 5 | cis | P.F.: 228 - 230°C dichlorhydrate |
| 49 | (3)Cl | H | 4 | 1 | 1 | 0 | 4 | cis | P.F.: 120 - 121°C base |
| 50 | (3)$CF_3$ | H | 4 | 1 | 1 | 0 | 4 | cis | P.F.: 112 - 114°C base |
| 51 | (3)Cl | H | 4 | 1 | 0 | 0 | 3 | | P.F.: 129°C base |
| 52 | (3)$CF_3$ | H | 4 | 1 | 0 | 0 | 3 | | P.F.: 109°C base |
| 53 | (3)Cl | H | 5 | 1 | 1 | 0 | 4 | cis | P.F.: 230 - 232°C dichlorhydrate |
| 54 | (3)$CF_3$ | H | 5 | 1 | 1 | 0 | 4 | cis | P.F.: 220°C dichlorhydrate |
| 55 | (3)Cl | H | 5 | 0 | 2 | 0 | 5 | cis | P.F.: 198 - 200°C dichlorhydrate |
| 56 | (3)$CF_3$ | H | 5 | 0 | 2 | 0 | 5 | cis | P.F.: 180 - 185°C dichlorhydrate |

**Etude pharmacologique des composés de la présente invention**

L'activité analgésique des composés de la présente invention a été recherchée au moyen du test de tail-flick selon D'Amour F.E. et Smith D.L., Journal of pharmacology and experimental therapeutics, 72, 74 - 79 (1941), du test de la plaque chauffante de Woolfe G. et Mac Donald A.D., Journal of pharmacology and experimental therapeutics, 80, 300 - 307 (1944), et du test à la phenylbenzoquinone d'après Siegmund E., Cadmus R. et Lu G, Proc. Soc. Exptle. Biol. Med. 95, 729 - 731 (1957).

L'activité anxiolytique a été recherchée de son côté, au moyen du test des quatre plaques d'Aron C., Simon P., Larousse C. et Boissier J.R., Neuropharmacology 10, 459 - 469, (1971), du test du conflit de Vogel J.R., Beer E., Clody D.E., Psychopharmacol. 21, 1 - 7, (1971) et du test de punition de Mac Millan D.E., J. Exp. Anal. Behav, 19, 133 - 145, (1973).

A titre d'exemple, le tableau suivant regroupe les résultats des effets analgésiques observés chez les souris pour quelques produits représentatifs de l'invention:

| Composés des Exemples N° | Test de Siegmund $DE_{50}$ mg/kg PO | Test Hot Plate $DE_{50}$ mg/kg IP | Test Tail-Flick $DE_{50}$ mg/kg IP |
|---|---|---|---|
| 2 | 8,7 | > 50 | > 50 |
| 3 | 16,0 | 19,8 | > 50 |
| 12 | 25,7 | 25,3 | 30,7 |
| 16 | 17,0 | 24,3 | > 50 |
| 17a | 5,82 | 21,3 | > 50 |
| 36 | 4,3 | $25 < DE_{50} < 50$ | > 50 |
| 40 | 7,5 | 53,9 | > 50 |
| 42 | 11,4 | | > 50 |

Note: La dose efficace moyenne ($DE_{50}$) a été calculée:

- dans le test de Siegmund, à partir des pourcentages de variation du nombre de contractions de l'abdomen par rapport au lot témoin;
- dans le test hot-plate, à partir des pourcentages de variation du temps de léchage (en secondes) par rapport au lot témoin.
- dans le test tail-flick, à partir des pourcentages de variation du temps de réaction (en secondes) par rapport au lot témoin.

Les composés des exemples 2, 36 et 40 présentent une activité analgésique très importante sur le test de Siegmund. Sur le test de tail-flick aucun composé n'est actif à la dose de 50 mg/kg IP; à l'exception du composé de l'exemple 12.

Sur le test de la plaque chauffante, les composés présentent une activité analgésique modérée puisque les $DE_{50}$ calculées sont en général comprises entre 20 et 50 mg/kg IP, ce qui montre que, pour les dérivés de l'invention, la composante centrale des effets analgésiques observés est faible.

Les effets anxiolytiques des composés de l'invention sont illustrés par les résultats répertoriés dans les tableaux suivants:

**Test des 4 plaques (souris)**

| | % Nombre des chocs recus (N = 10) | | | |
|---|---|---|---|---|
| Doses Mg/kg IP → | 2,5 | 5 | 10 | 25 |
| Composé de l'exemple N° | | | | |
| 3 | + 38 % S | + 44 % S | + 48 % S | + 18 % |
| 12 | + 38 % S | + 15 % | + 20 % | + 2 % |
| 16 | - 12 % | + 2 % | + 32 % S | + 5 % |
| 17a | + 18 % | + 26 % S | + 35 % S | + 61 % S |
| 17b | + 15 % | 34 % S | + 53 % S | + 27 % |
| 36 | - 12 % | - 4 % | - 4 % | + 39 % S |

S = Modification significative $p < 0,05$ des chocs reçus.

**Test de Mac-Millan conditionnement opérant de punition (rats)**

**Pourcentage de variation du nombre de reponses punies (N > 9)**
(N = Nombre d'animaux)

| Doses Mg/kg IP → | 2,5 | 5 | 10 | 20 |
|---|---|---|---|---|
| Composé de l'exemple N° | | | | |
| 12 | - 33,5 % | - 24 % S | - 51,3 % | - 75 % S |
| 16 | + 5,4 % | + 42,2 % | + 125,9 % S | - 60,7 % S |
| 17a | + 47,8 % S | + 33,6 % S | + 42,6 % | - 70,3 % S |

S = Modification significative.

Sur le test des 4 plaques, les composés des exemples 3, 12, 16, 17a, 17b et 36 fournissent des résultats en faveur d'une activité anxiolytique très importante. Il en est de même, sur le test de conflit de Mac Millan, pour les composés des exemples 16 et 17a.

Ce qui permet de conclure à une utilisation thérapeutique très intéressante des produits de l'invention dans le traitement des états anxieux d'origine endogène.

**Revendications** pour les Etats Contractants: BE, CH, DE, FR, GB, IT, LT, LU, NL, SE

1. Les dérivés du triazole de formule générale I:

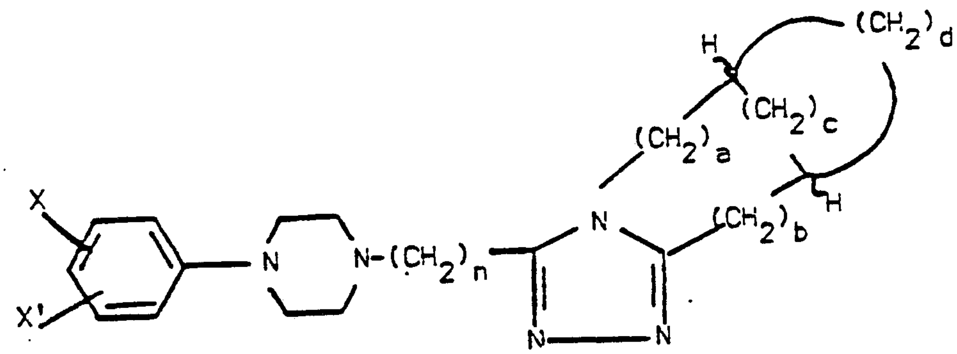

(I)

dans laquelle:

- X et X', identiques ou différents, représentent chacun un atome d'hydrogène ou d'halogène, un radical trifluorométhyle ou un radical alcoyle ou alcoxy ayant chacun de 1 à 5 atomes de carbone ou
- X et X' représentent ensemble un radical méthylène dioxy;
- n représente un nombre entier de 1 à 6;
- a représente les valeurs zéro, 1 ou 2;
- b représente les valeurs zéro, 1, 2 ou 3;
- c représente les valeurs zéro, 1 ou 2, et
- d'représente les valeurs, 2, 3, 4 ou 5 de telle sorte que la somme; a + b + c + d soit égale à 4, 5, 6 ou 7.

2. Les sels des composés de la revendication 1 avec des acides appropriés.

3. Les sels selon la revendication 2, qui sont physiologiquement tolérables.

4. Le β-N'-[N-(métachlorophényl) pipérazine] éthyl-9 [7H] hexahydro-2b,3,4,5,6,6a triazolo [3,4-a] isoindole.

5. La cis N-[N'-(métatrifluorométhylphényl) pipérazine] n.propyl-10 octahydro-3,3a,4,5,6,7,7a,8 triazolo [4,3-b] isoquinoléine.

6. La trans β-N-[N'(métachlorophényl) pipérazine] éthyl-10 octahydro-3,4,4a,5,6,7,8,8a triazolo [4,3-a] quinoléine.

7. La cis β-N-[N'(métatrifluorométhylphényl) pipérazine] éthyl-10 octahydro-3,3a,4,5,6,7,7a,8 triazolo [4,3-b] isoquinoléine.

8. Le β-N-[N'-(métatrifluorométhylphényl) pipérazine] éthyl-8 [6-H] dihydro-2b, 5a triazolo [3,4-a] cyclopenta (c) pyrrole, son méthane sulfonate, son monochlorhydrate et son dichlorhydrate.

9. Le β-N-[N'-(métatrifluorométhylphényl)] pipérazine éthyl-9 [7H] hexahydro-2b,3,4,5,6,6a triazolo- [3,4-a] isoindole.

10. La cis γ-N-[N'-(métachlorophényl) pipérazine] n.propyl-10 octahydro-3,3a,4,5,6,7,7a,8 triazolo [4,3-b] isoquinoléine.

11. Le procédé de préparation des composés de la revendication 1 caractérisé en ce que:
- l'on fait réagir un composé de formule générale II:

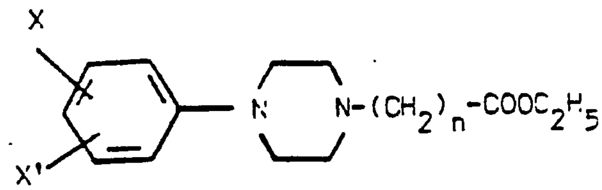

(II)

dans laquelle X, X' et n ont les significations énoncées dans la revendication 1, avec l'hydrazine ($H_2N$-$NH_2$) pour obtenir un composé de formule générale III

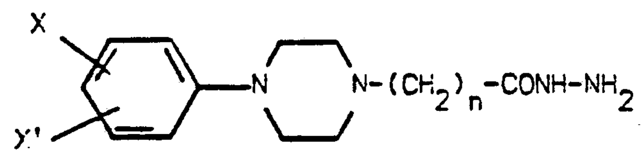

(III)

dans laquelle X, X' et n sont tels que définis dans la revendication 1,
- et l'on fait réagir ce composé (III) avec un lactime éther de formule générale IV:

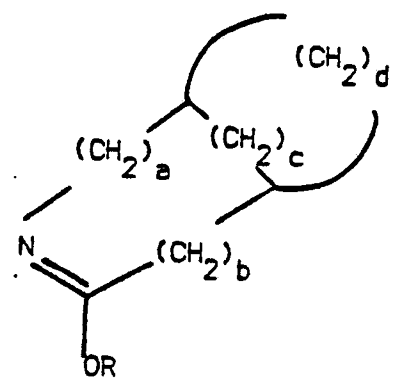

(IV)

dans laquelle a, b, c, et d'ont les significations définies dans la revendication 1, et R représente un radical alcoyle ayant de 1 à 5 atomes de carbone.

12. Les compositions pharmaceutiques contenant comme principe actif un composé selon les revendications 1 et 3 à 10, avec des excipients pharmaceutiques appropriés.

13. Les compositions pharmaceutiques selon la revendication 12 présentées sous une forme convenant notamment pour le traitement de la douleur et des états anxieux.

**Revendication** pour l'Etat Contractant: AT

Procédé de préparation des dérivés du triazole de formule générale I:

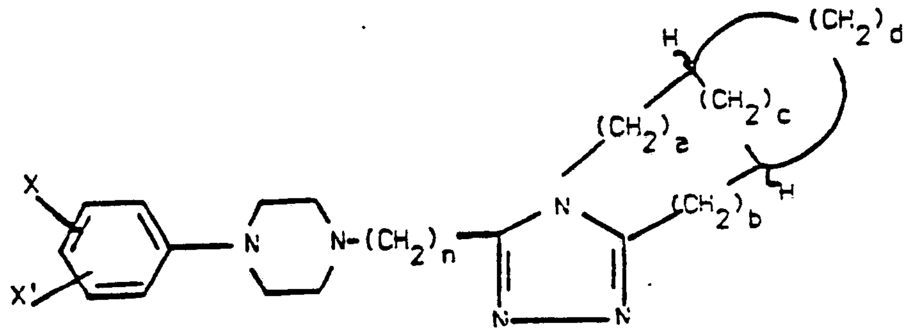

(I)

dans laquelle:

- X et X', identiques ou différents, représentent chacun un atome d'hydrogène ou d'halogène, un radical trifluorométhyle ou un radical alcoyle ou alcoxy ayant chacun de 1 à 5 atomes de carbone ou
- X et X' représentent ensemble un radical méthylène dioxy;
- n représente un nombre entier de 1 à 6;
- a représente les valeurs zéro, 1 ou 2;
- b représente les valeurs zéro, 1, 2 ou 3;
- c représente les valeurs zéro, 1 ou 2, et
- d représente les valeurs 2, 3, 4 ou 5 de telle sorte que la somme; a + b + c + d soit égale à 4, 5, 6 ou 7.
- et de leurs sels d'addition avec des acides appropriés;

caractérisé en ce que:

- l'on fait réagir un composé de formule générale II:

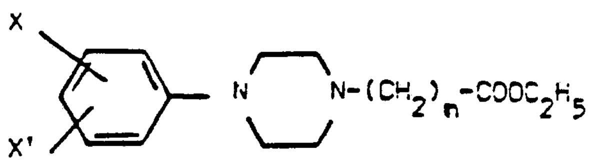

(II)

dans laquelle X, X' et n ont les significations énoncées ci-dessus,
avec l'hydrazine ($H_2N$-$NH_2$) pour obtenir un composé de formule générale III

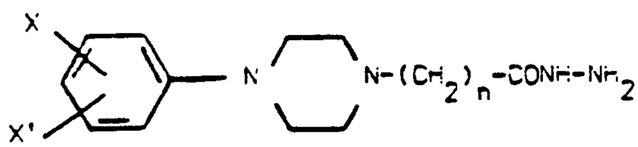

(III)

dans laquelle X, X' et n sont tels que définis précédemment,

- et l'on fait réagir ce composé (III) avec un lactime éther de formule générale IV:

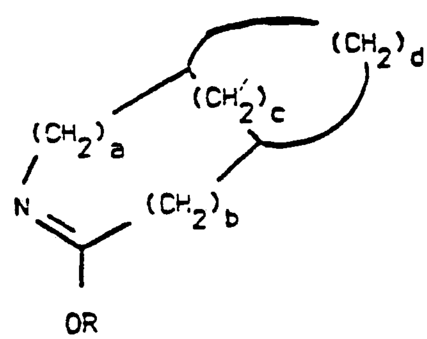

(IV)

dans laquelle a, b, c, et d ont les significations définies précédemment, et R représente un radical alcoyle ayant de 1 à 5 atomes de carbone;

et si on le désire, on traite les dérivés ainsi obtenus, avec des acides appropriés pour donner les sels d'addition correspondants.

**Patentansprüche** für die Vertragsstaaten: DE, CH, BE, FR, GB, IT, LI, LU, NL, SE

1. Triazolderivate der allgemeinen Formel I

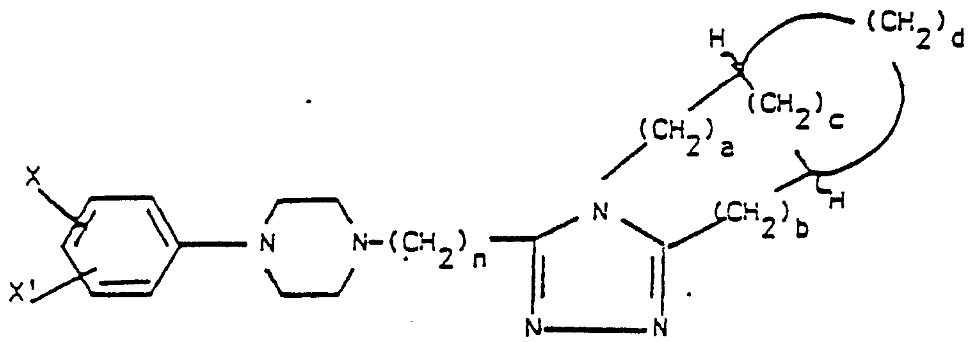

(I)

in der

- X und X', die gleichartig oder verschieden sein können, jeweils Wasserstoffatome oder Halogenatome. Trifluormethylgruppen oder Alkyl- oder Alkoxygruppen mit jeweils 1 bis 5 Kohlenstoffatomen oder
- X und X' gemeinsam eine Methylendioxygruppe;
- n eine ganze Zahl mit einem Wert von 1 bis 6;
- a eine Zahl mit einem Wert von 0, 1 oder 2;
- b eine Zahl mit einem Wert von 0, 1, 2 oder 3;
- c eine Zahl mit einem Wert von 0, 1 oder 2, und
- d eine Zahl mit einem Wert von 2, 3, 4 oder 5 mit der Maßgabe, daß die Summe $a + b + c + d$ gleich 4, 5, 6 oder 7 ist.

2. Die Salze der Verbindungen von Anspruch 1 mit geeigneten Säuren.

3. Die Salze nach Anspruch 2, die physiologisch verträglich sind.

4. 9-{β-N'-[N-(m-Chlorphenyl)-piperazin]-ethyl}-[7H]-2b,3,4,5,6,6a-hexahydrotrinzolo[3,4-a]-isoindol.

5. 10-{cis-N-[N'-(m-Trifuormethylphenyl)-piperazin]-n-propyl}-3,3a,4,5,6,7,7a,8-octahydro-triazolo-[4,3-b]isochinolin.

6. 10-{trans-β-N-[N'-(m-Chlorphenyl)-piperazin]-ethyl}3,4,4a,5,6,7,8,8a, octahydro-triazolo]4,3-alchinolin.

7. 10-{cis-β-N-[N'-(m-Trifuormethylphenyl)-piperazin]-ethyl}-3,3a,4,5,6,7,7a,8-octahydro-triazolo[4,3-b]isochinolin.

8. 8-{β-N-[N'-(m-Trifuormethylphenyl)-piperazin]-ethyl}-[6-H]-2b,5a-dihydrotriazolo[3,4-a]-cyclopenta(c)pyrrol, dessen Methansulfonat, dessen Monochlorhydrat und dessen Dichlorhydrat.

9. 9-{β-N-[N'-(m-Trifuormethylphenyl)-piperazin]- ethyl}-[7H]-2b,3,4,5,6,6a-hexahydro-triazolo [3,4-a]isoindol.

10. 10-{cis-γ-N-[N'-(m- Chlorphenyl)-piperazin]-n-propyl}-3,3a,4,5,6,7,7a,8-octahydro-triazolo [4,3-b]isochinolin.

11. Verfahren zur Herstellung der Verbindungen nach Anspruch 1, dadurch gekennzeichnet, daß man eine Verbindung der allgemeinen Formel II

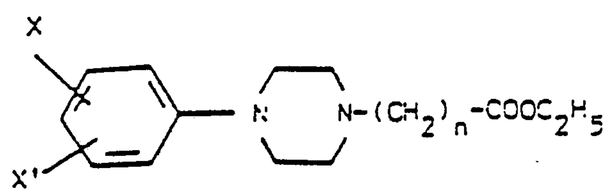

(II)

worin X, X' und n die in Anspruch 1 angegebenen Bedeutungen besitzen, mit Hydrazin ($H_2H\text{-}NH_2$) zur Bildung einer Verbindung der allgemeinen Formel III

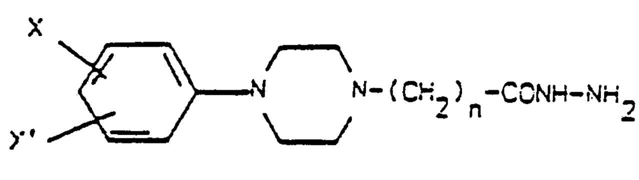

(III)

in der X, X' und n die in Anspruch 1 angegebenen Bedeutungen besitzen, umsetzt und

- die Verbindung (III) mit einem Lactimether der allgemeinen Formel IV

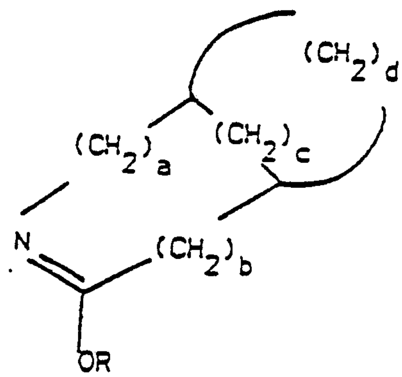

(IV)

worin a, b, c und d die in Anspruch 1 angegebenen Bedeutungen besitzen und R eine Alkylgruppe mit 1 bis 5 Kohlenstoffatomen darstellt, umsetzt.

12. Pharmazeutische Zubereitungen enthaltend als Wirkstoff eine Verbindung nach einem der Ansprüche 1 und 3 bis 10 in Kombination mit geeigneten pharmazeutischen Trägermaterialen.

13. Pharmazeutische Zubereitung nach Anspruch 12 in einer insbesondere zur Behandlung von Schmerzen und von Angstzuständen geeigneten Form.

**Patentanspruch** für den Vertragsstaat: AT

Verfahren zur Herstellung von Triazolderivaten der allgemeinen Formel I

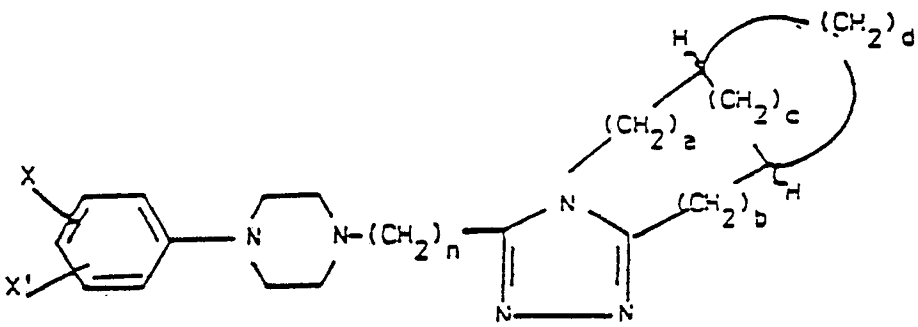

(I)

in der

- X und X', die gleichartig oder verschieden sein können, jeweils Wasserstoffatome oder Halogenatome, Trifluormethylgruppen oder Alkyl- oder Alkoxygruppen mit jeweils 1 bis 5 Kohlenstoffatomen oder
- X und X' gemeinsam eine Methylendioxygruppe;
- n eine ganze Zahl mit einem Wert von 1 bis 6;
- a eine Zahl mit einem Wert von 0, 1 oder 2;
- b eine Zahl mit einem Wert von 0, 1, 2 oder 3;
- c eine Zahl mit einem Wert von 0, 1 oder 2, und
- d eine Zahl mit einem Wert von 2, 3, 4 oder 5 mit der Maßgabe, daß die Summe $a + b + c + d$ gleich 4, 5, 6 oder 7 ist,
- und deren Additionssalzen mit geeigneten Säuren, dadurch gekennzeichnet, daß man eine Verbindung der allgemeinen Formel II

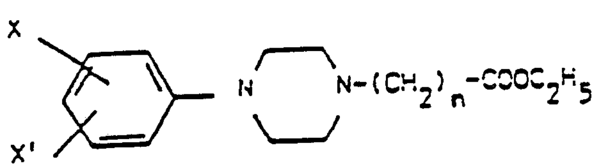

(II)

worin X, X' und n die oben angegebenen Bedeutungen besitzen, mit Hydrazin ($H_2H$-$NH_2$) zur Bildung einer Verbindung der allgemeinen Formel III

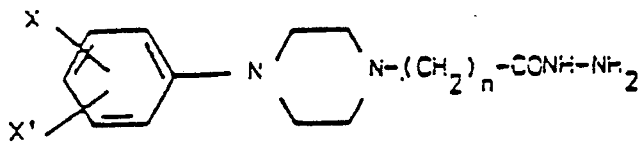

(III)

in der X, X' und n die oben angegebenen Bedeutungen besitzen, umsetzt und
- die Verbindung (III) mit einem Lactimether der allgemeinen Formel IV

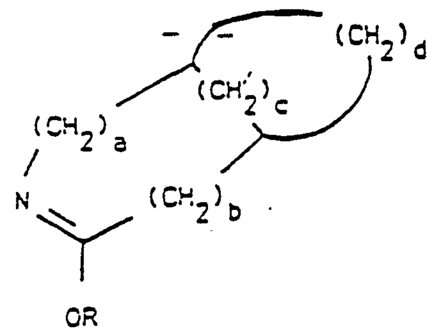

(IV)

worin a, b, c und d die oben angegebenen Bedeutungen besitzen und R eine Alkylgruppe mit 1 bis 5 Kohlenstoffatomen darstellt, umsetzt; und

gewüschtenfalls die in dieser Weise erhaltenen Derivate mit geeigneten Säuren zu den entsprechenden Additionssalzen umsetzt.

**Claims** for the contracting states: BE, CH, DE, FR, GB, IT, LI, LU, NL, SE

1. Triazole derivatives of the general formula I:

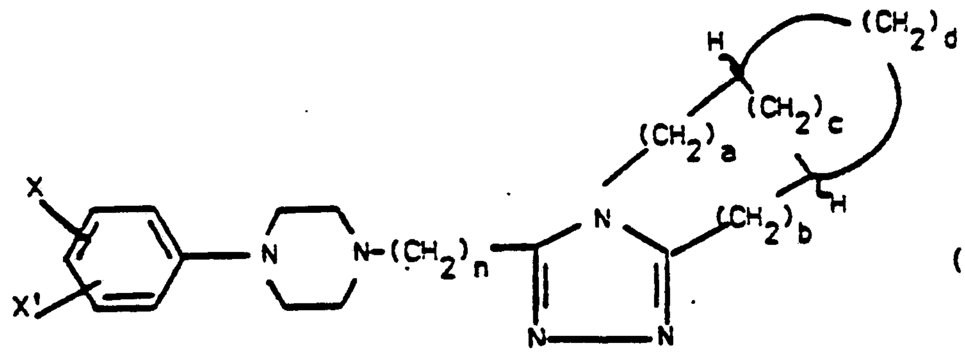

(I),

in which
- X and X', which are the same or different, each represents a hydrogen or halogen atom, a trifluoromethyl radical, or an alkyl or alkoxy radical each having from 1 to 5 carbon atoms, or
- X and X' together represent a methylenedioxy radical,
- n represents an integer of from 1 to 6,
- a represents the value 0, 1 or 2,
- b represents the value 0, 1, 2 or 3,
- c represents the value 0, 1 or 2, and
- d represents the value 2, 3, 4 or 5 such that the sum of a + b + c + d is equal to 4, 5, 6 or 7.

2. The salts of the compounds of claim 1 with appropriate acids.

3. The salts according to claim 2 that are physiologically tolerable.

4. 9-{β-N'-[N-(metachlorophenyl)piperazine]ethyl}-[7H]-2b,3,4,5,6,6a-hexahydrotriazolo[3,4-a]isoindole.

5. Cis-10-{N-[N'-(metatrifluoromethylphenyl)piperazine]n.propyl}-3,3a,4,5,6,7,7a,8-octahydrotriazolo[4,3-b]isoquinoline.

6. Trans-10-{β-N-[N'-(metachlorophenyl)piperazine]ethyl}-3,4,4a,5,6,7,8,8a-octahydrotriazolo[4,3-a]quinoline.

7. Cis-10-{α-N-[N'-(metatrifluoromethylphenyl)piperazine]ethyl}-3,3a,4,5,6,7,7a,8-octahydrotriazolo[4,3b]iso quinoline.

8. 8-{α-N-[N'-(metatrifluoromethylphenyl)piperazine]ethyl}-[6-H]-2b,5a-dihydrotriazolo[3,4-a]cyclopenta(c)pyrrole, its methanesulphonate, monohydrochloride and dihydrochloride.

9. 9-{α-N-[N'-(metatrifluoromethylphenyl)piperazine]ethyl}-[7H]-2b,3,4,5,6,6a-hexahydrotriazolo[3,4a]iso indole.

10. Cis-10-{γ-N-[N'-(metachlorophenyl)piperazine]n.propyl}-3,3a,4,5,6,7,7a,8-octahydrotriazolo[4,3b]iso quinoline.

11. The process for the preparation of the compounds of claim 1, characterised in that:

- a compound of the general formula II

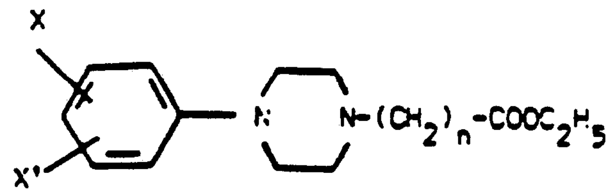

(II)

in which X, X' and n have the meanings defined in claim 1 is reacted with hydrazine ($H_2N-NH_2$) to obtain a compound of the general formula III

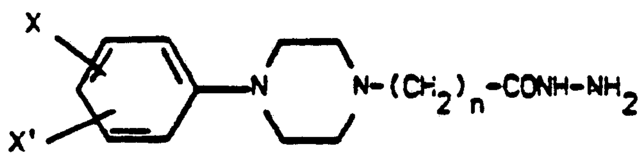

(III)

in which X, X' and n are as defined in claim 1,

- then, this compound (III) is reacted with a lactime ether of the general formula IV

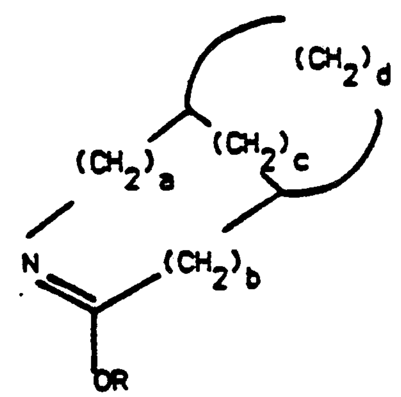

(IV)

in which a, b, c and d are as defined in claim 1 and R represents an alkyl radical having from 1 to 5 carbon atoms.

12. Pharmaceutical compositions containing as active ingredient a compound according to claims 1 and 3 to 10 together with appropriate pharmaceutical excipients.

13. Pharmaceutical compositions according to claim 12 presented in a form suitable especially for the treatment of pain and anxiety.

**Claim** for the contracting state: AT

Process for the preparation of triazole derivatives of the general formula I:

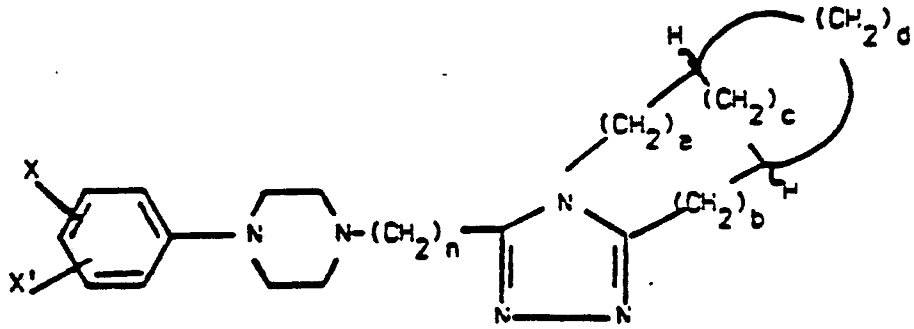

(I),

in which

- X and X', which are the same or different, each represents a hydrogen or halogen atom, a trifluoromethyl radical, or an alkyl or alkoxy radical each having from 1 to 5 carbon atoms, or
- X and X' together represent a methylenedioxy radical,
- n represents an integer of from 1 to 6,
- a represents the value 0, 1 or 2,
- b represents the value 0, 1, 2 or 3,
- c represents the value 0, 1 or 2, and
- d represents the value 2, 3, 4 or 5 such that the sum of a + b + c + d is equal to 4, 5, 6 or 7,

and the addition salts thereof with appropriate acids,
characterised in that:

- a compound of the general formula II

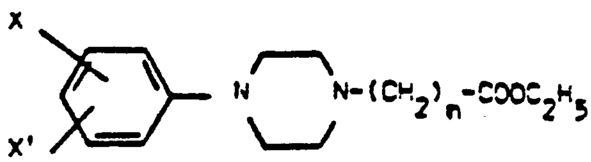

(II)

in which X, X' and n have the meanings defined above is reacted with hydrazine ($H_2N-NH_2$) to obtain a compound of the general formula III

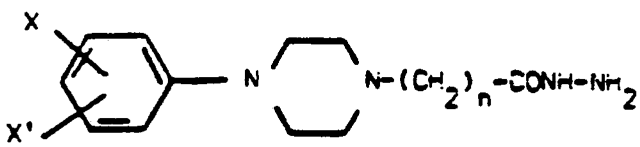

(III)

in which X, X' and n are as defined above,

- then, this compound (III) is reacted with a lactime ether of the general formula IV

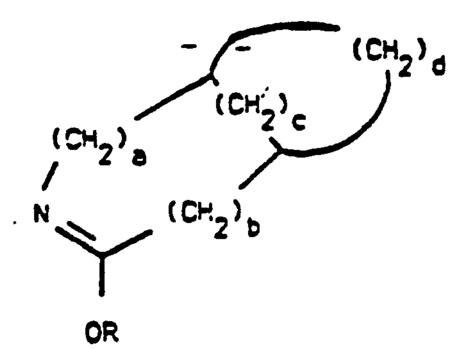

(IV)

in which a, b, c and d are as defined above and R represents an alkyl radical having from 1 to 5 carbon atoms, and, if desired, the derivatives so obtained are treated with appropriate acids to give the corresponding addition salts.